# NEW EUROPEAN PATENT SPECIFICATION

(11) **EP 1 255 449 B2**
(45) Date of publication and mention of the opposition decision: **08.06.2016**
(45) Mention of the grant of the patent: 28.03.2007
(21) Application number: 01910704.4
(22) Date of filing: 15.02.2001
(51) Int. Cl.: A23C 9/20

(54) **NUTRITIONAL FORMULATION CONTAINING PREBIOTIC SUBSTANCES**
PREBIOTISCHE SUBSTANZEN ENTHALTENDE ERNÄHRUNGSZUSAMMENSETZUNGEN
FORMULATION NUTRITIONNELLE CONTENANT DES SUBSTANCES PREBIOTIQUES

(30) Priority: 17.02.2000 US 506009
(43) Date of publication of application: 13.11.2002
(73) Proprietor: Nestec S.A., 1800 Vevey (CH)
(72) Inventor: WILSON, Jeffrey, L., Doylestown, Pennsylvania 18901 (US)
(74) Representative: Rupp, Christian
(86) International application number: PCT/US2001/004794
(87) International publication number: WO 2001/060346

(56) References cited:
- WO-A-00/10402
- WO-A-94/18986
- WO-A-97/02830
- WO-A-98/31241
- WO-A-99/64022
- US-A- 5 514 660
- US-A- 5 827 526
- DATABASE WPI Section Ch, Week 199751 Derwent Publications Ltd., London, GB; Class D13, AN 1997-550635 XP002171552 & CN 1 129 524 A (SONGHUAJIANG NO 2 MILK PROD FACTORY HARB), 28 August 1996 (1996-08-28)
- IDOTA T. ET AL: 'Growth-promoting Effects of N-Acetylneuraminic Acid-containing Substances on Bifidobacteria' BIOSCI. BIOTECH. BIOCHEM. vol. 58, no. 9, 1994, pages 1720 - 1722

## Description

### FIELD OF THE INVENTION

This invention relates to the use of nutritional formulations containing prebiotic substances in the growth promotion of beneficial microorganisms and the inhibition of pathogenic organisms. More specifically, this invention relates to the use of nutritional formulations containing oligofructose and sialyllactose.

### BACKGROUND OF THE INVENTION

Oligofructose is a series of natural oligosaccharides found primarily in vegetables, such as onion and the root of the chicory plant. Oligofructose is known to be a specific substrate for Bifidobacteria. (See, e.g., Mitsuoka et al, "Effect of Fructo-oligosaccharides on Intestinal Microflora", Die Nahrung, 3, 5-6: 427-436 (1987))

Oligofructose passes through the small intestine without being digested, reaching the large intestine. In the large intestine, oligofructose is fermented only by a limited range of microorganisms that include most species of Bifidobacteria, i.e., species of bacteria beneficial for human health. (See Bouhnik et al, "Short Chain Fructo-Oligosaccharide Administration Dose-Dependently Increases Fecal Bifidobacteria in Healthy Humans," J. Nutrition, 129:113-116)

For example, oligofructose can be utilized efficiently by Lactobacilli and Bifidobacteria. It is known that in mixed populations of bacteria such as that which exists in the human colon, oligofructose is consumed preferentially by Bifidobacteria. The other bacteria present in this "mixed population" either do not grow or are inhibited from growing. (See, e.g., Gibson et al, "Selective Stimulation of Bifidobacteria in the Human Colon, by oligofructose and Insulin." Gastroenterol, 108:975-982 (1995))

Moreover, it is known that a metabolic by-product of Bifidobacteria is short chain fatty acids, resulting in a reduction of the pH in the digestive tract. This pH effect has been observed clinically and documented in Mitsuoka et al, "Effect of Fructo-oligosaccharides on Intestinal Microflora", Die Nahrung, 3,5-6: 427 - 438 (1987).

Sialyllactoses are oligosaccharides which occur naturally in human milk as well as in milk of other mammals. However, sialyllactoses are present at noticeably higher concentrations in human milk compared to other mammalian species.

The two primary species of sialyllactose are 3'-sialyllactose and 6'-sialyllactose. These species occur naturally in human milk at a relative ratio of 1:3 (3':6'). Sialyllactose is known to have anti-adhesive properties for specific pathogenic bacteria. For example, it has been suggested that sialyllactose acts to inhibit cholera toxin (see, Idota et al, "Inhibition of Cholera Toxin by Human Milk Fractions and Sialyllactose," Biosci. Biotech. Biochem. 59:417-419) and Helicobacter pylori (see, Simon et al, "Inhibition of Helicobacter pylori Binding to Gastrointestinal Epithelial Cells by Sialic Acid-Containing Oligosaccharides," Infection and Immunity, 750-757, (1997)). In light of its antiadhesive properties, sialyllactose has been used to treat a number of medical conditions. For example, U.S. Patent Nos. 5,514,660 and 5,753,630, describe the use of sialyllactose in the treatment and inhibition of duodenal ulcers. U.S. Patent No. 5,883,079 describes the use of sialyllactose to inhibit H. pylori infection in mammalian tissue.

However, the use of two prebiotic substances, specifically, oligofructose and sialyllactose, in combination has heretofore not been described. Accordingly, it can be seen that there is a need for such a combination.

### SUMMARY OF THE INVENTION

The present invention relates to the use according to claim 1.

### BRIEF DESCRIPTION OF THE DRAWING

Fig 1 shows the growth of Bifidobacteria, Clostridia and Bacteroides as set forth in Example 1.
Fig 2 shows the growth of B. lactis as set forth in Example 2.
Fig 3 shows the growth of Clostridia, Bacteroides and B. lactis as shown in Example 3.

### DETAILED DESCRIPTION OF THE INVENTION

The sialyllactose useful in the present compositions comprises a mixture of 3'-siallylactose and 6'-siallyllactose. Preferably, the sialyllactose used herein is 3'-siallylactose. The sialyllactose useful in the present compositions may be prepared according to any of the methods described, e.g., in U.S. Patent No. 5,575,916; 5,714,075; 5,278,299; 5,374,541; and 5,876,980. However, it will be recognized by those skilled in the art that any other known method of synthesizing and purifying sialyllactose may be used to prepare the sialyllactose, useful in the present compositions.

The oligofructose component of the present composition may be prepared from a naturally occurring polyfructose (inulin) which may be found in many plants, including onions, leeks, wheat, chicory and artichoke. Chicory is most commonly used. Oligofructose can be recovered in sufficient quantities, from these plants, by methods known in the art. The naturally occurring inulin comprises oligofructose and higher polymers of fructose. The inulin can be separated as it is soluble in hot water. If desired, the naturally occurring oligofructose can be separated as it is additionally soluble in cold water. The inulin, optionally after removal of the naturally occurring oligofructose, may be converted into oligofructose by hydrolysis. The inulin may be broken down into oligomeric chain lengths by using an inulase enzyme. Inulin can also be degraded into oligomeric chain lengths by chemical hydrolysis. The oligofructose component of the present composition may also be prepared by synthesis rather than by extraction procedures. Oligofructose may be synthesized from sucrose by transfructosylation, which is accomplished by means of an enzyme, β-fructofuranosidase, that links additional fructose monomers to the sucrose molecule. Oligofructose formed in this manner contains fructose units linked to a terminal glucose unit. Oligofructose derived from inulin from plants such as chicory contains both fructose chains and fructose chains with a terminal glucose unit'. Oligofructose prepared by methods such as these is commercially available. A preferred form of oligofructose for purposes of this invention is Raftilose® available from Orafti S.A., Tienen, Belgium. Again, it is understood that any known method of synthesizing and/or isolating oligofructose may be suitable for the present invention.

The nutritional composition contains 0.3 g/L to 6 g/L of oligofructose and 60 mg/L to 1 g /L of sialyllactose, more preferably 1 g/L to 3 g/L of oligofructose and 100 mg/L to 600 ml/L of sialyllactose and even more preferably about 3 g/L of oligofructose and about 100 mg/L of sialyllactose.

The present inventors have found that the combination of oligofructose and sialyllactose in the present nutritional formulations produces a synergistic prebiotic effect, i.e., a prebiotic effect greater than the additive effect of the substances alone. The present use of oligofructose and sialyllactose specifically increases the concentration of beneficial bacteria (Bifidobacteria) in the gut while having no effect on pathogenic bacteria (e.g., Clostridia, Bacteroides, E. coli, etc). In addition, although the administration of oligofructose is known to lower the pH of its environment, the combination of oligofructose with sialyllactose has been found to lower the pH to an even greater extent. This reduction in gut pH results in an environment which is less conducive to the growth of certain organisms, specifically, the less beneficial or more pathogenic bacteria such E. coli or Bacteroides.

The nutritional compositions can be utilized in conjunction with various nutritional products, such as infant formula, follow-on formula, toddler's beverage, milk, yogurt, fruit-based products for older children (such as fruit juices) candies, chewing gum, lozenges, powders, tablets, etc. Preferably the present compositions are added to infant formula. The infant formula can be in the form of a ready to feed liquid or a powder, which may be mixed with water and fed to the infant. It is most preferred that the present formulation be added to infant formula in liquid form.

Infant formula suitable for use with the present invention should contain all vitamins and minerals considered to be essential in the daily diet. These vitamins and minerals should be present in nutritionally significant amounts. Examples of vitamins, minerals and other nutrients which may be included in infant formulas in which the present formulations are to be added include vitamin A, vitamin B complex, vitamin C, vitamin D, vitamin E, vitamin K, calcium, magnesium sodium, potassium, phosphorous, copper, zinc, chloride, iodine, selenium, iron, niacin, folic acid, pantothenic acid, biotin, chlorine, Inositol and manganese.

The infant formula may contain one or more lipid sources as will be recognized by those skilled in the art. The infant formula may further contain other substances than to have a beneficial effect, such as, nucleotides, immunoglobulins, polyunsaturated fatty acids, etc.

A preferred infant formula is as follows:

| Ingredient | Units | Per Liter |
|---|---|---|
| Energy | Kcal | 672 |
| Protein | g | 15 |
| Whey: Casein ratio | | 60:40 |
| Fat | g | 36 |
| Carbohydrate | g | 72 |
| Oligofructose | g | 3.0 |
| Sialyllactose | mg | 100 |
| Vitamin A | RE | 750 |
| Mixed natural carotids | IU | 400 |
| Vitamin D | mcg | 10.6 |
| Vitamin F | mg | 7.4 |
| Vitamin K | mcg | 67.0 |
| Vitamin B₁ (thiamin) | mcg | 1000 |
| Vitamin B₂ (riboflavin) | mcg | 1500 |
| Vitamin B₆ (pyridoxine) | mcg | 600 |
| Vitamin B₁₂ (cyanacobalmine) | mcg | 2.0 |
| Niacin | mcg | 9.0 |
| Folic Acid | mcg | 80 |
| Pantothenic Acid | mcg | 3000 |
| Biotin | mcg | 90 |
| Vitamin C (ascorbic acid) | mg | 90 |
| Choline | mg | 100 |
| Inositol | mg | 33 |
| Calcium | Mg | 460 |
| Phosphorous | Mg | 333 |
| Magnesium | Mg | 64 |
| Iron | Mg | 8.0 |
| Zinc | Mg | 6.0 |
| Manganese | mcg | 50 |
| Copper | mcg | 560 |
| Iodine | mcg | 100 |
| Sodium | mg | 160 |
| Potassium | mg | 650 |
| Chloride | mg | 433 |
| Selenium | mcg | 14 |

The present invention is further described with reference to the following examples:

### Example 1

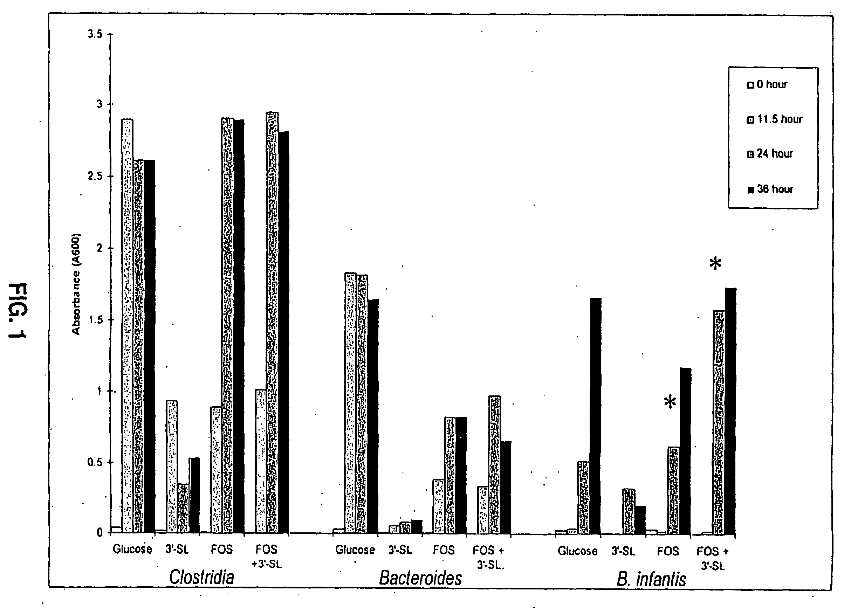

Identical inoculua (A₆₀₀ = 1.0) of overnight cultures (PYG broth, 37° C, anaerobic) of each organism (Clostridia, Bacteroides, or B. lactis) were grown in batch culture (37° C, anaerobic) with constant stirring. Carbohydrate-deficient batch culture media (PYG) was supplemented with either glucose (3.0 g/L), oligofructose (FOS at 3.0 g/L), sialyllactose (3'- or 6'-sialyllactose at 100 ml/L), or oligofructose plus sialyllactose (3.0 g/l and 100 mg/L, respectively). Growth was monitored at various time points (see Figure 1) by determining the titers of the bacteria on selective agar (BIM-25 agar for Bifidobacteria and Wilkens-Chalgren agar for Clostridia and Bacteroides). Results of the growth analysis are shown in Figure 1.

As shown in Figure 1, substituting 3'-sialyllactose for glucose in the growth media resulted in essentially no growth of Clostridia or Bacteroides in comparison to glucose. However, the growth of Clostridia and B. infantis, but not Bacteroides, in the presence of oligofructose was similar to that seen in the presence of glucose. The combination of oligofructose and sialyllactose had no effect on the growth of Clostridia or Bacteroides. However, there was a noticeable effect on the growth of B. infantis, especially at the twenty four hour time point. It can be seen that the combination of oligofructose and sialyllactose had synergistic effect on the growth of B. infantis.

### Example 2

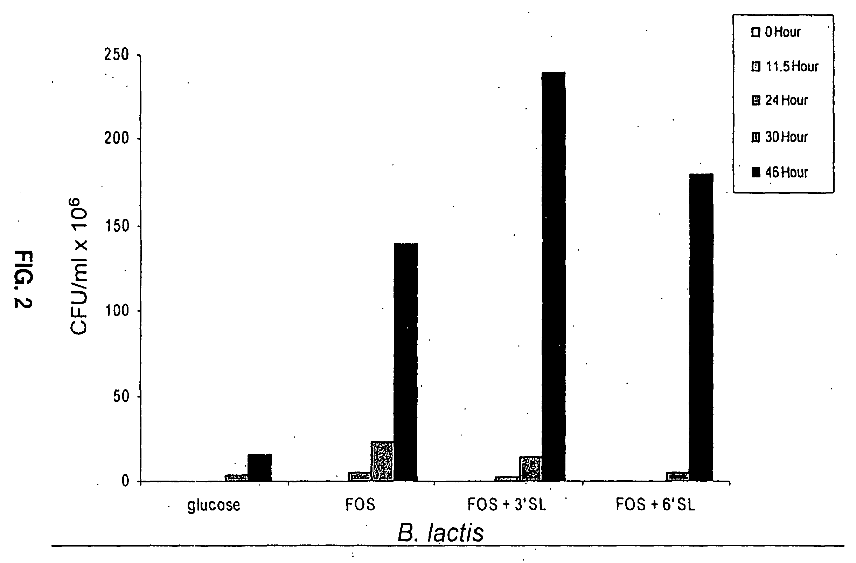

Identical inoculua (A₆₀₀ = 1.0) of overnight cultures (PYG broth, 37° C. anaerobic) of B. Lactis were grown in batch culture (37° C anaerobic) with constant stirring. Carbohydrate-deficient batch culture media (PYG) was supplemented with either glucose (3.0 g/L), oligofructose (3.0 g/L), or oligofructose (3.0g/L) plus sialyllactose (3'- or 6'-sialyl-lactose at 100 ml/L). Growth was monitored at various time points (see Figure 2) by determining the titers of the B. lactis bacteria on selective agar (BIM-25 agar). Results of the growth analysis are shown in Figure 2.

Figure 2 demonstrates that B. lactis grew significantly better in the presence of oligofructose than glucose. However, the growth of Bifidobacteria lactis was significantly enhanced in the presence of the present combination of oligofructose and either 3'- or 6'- sialyllactose at 46 hours.

### Example 3

A metabolic by-product of Bifidobacteria is short-chain fatty acids, the presence of which lowers the intestinal pH.

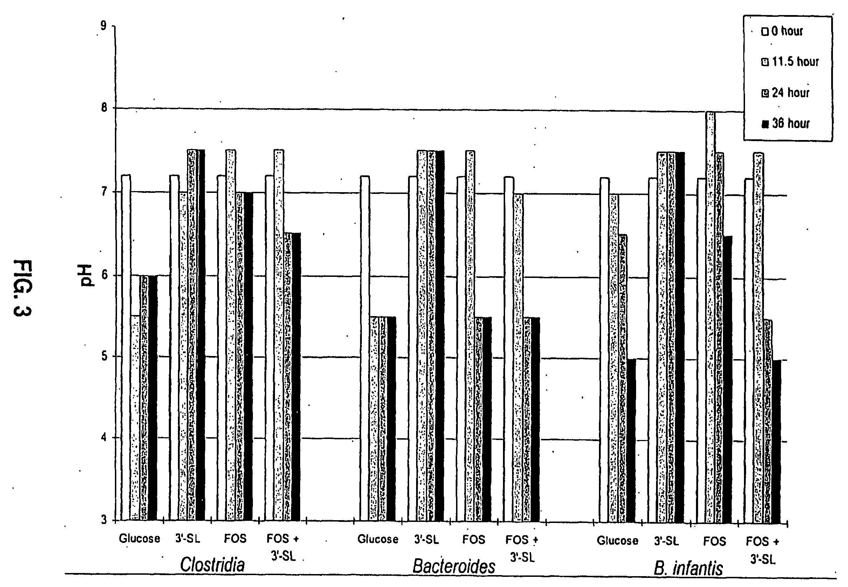

Figure 3 shows an analysis at different times post-inoculation of batch cultures of Clostridia, Bacteroides, and B. lactis. Identical inoculua (A ₆₀₀= 1.0) of overnight cultures (PYG broth, 37°C, anaerobic) of each organism was grown in batch culture (37°C, anaerobic) with constant stirring. Carbohydrate-deficient batch culture media (PYG) was supplemented as above. The media pH were monitored at various time points. Results of the pH analysis are shown in Figure 3.

As can be seen in Figure 3, in the presence of glucose, all three colonic bacteria lowered the pH. In the presence of sialyllactose alone, the pH increased in all cases. This is consistent with the poor microbial growth observed in the presence of sialyllactose alone. In the presence of oligofructose, the pH lowered, but to a lesser extent than glucose in batch cultures of Clostridia and B. infantis, but was similar to the pH change observed in the Bacteroides culture with glucose. In the presence of the combination of oligofructose and sialyllactose of the present invention, the pH lowered to a greater extent than with glucose.

However, this reduction in pH was less than that seen with glucose (Clostridia) or similar to the glucose pH change (Bacteroides and B. lactis). Of particular interest is the observation that while oligofructose alone shows some pH reduction over time in the bifidobacteria culture, the presence of sialyllactose dramatically reduced the pH compared to the oligofructose-only B. infantis culture. Thus, the synergistic effects of oligofructose and sialyllactose are present.

The present invention may be embodied in their specific forms without departure from the spirit or essential attributes thereof and, accordingly, reference should be made to the appended claims, rather than to the foregoing specification as indicating the scope of the invention.

## Claims

1. Use of oligofructose and sialyllactose in the preparation of a medicament for use as a nutritional composition for feeding to a human for increasing the amount of *Bifidobacteria* and inhibiting the binding of pathogenic bacteria in said human, wherein said nutritional composition is for feeding to a human at a concentration containing 0.3 g/L to 6 g/L of oligofructose and 60 mg/L to 1g/L of sialyllactose.

2. Use according to claim 1, wherein said concentration contains 1 g/L to 3 g/L of oligofructose and 100 mg/L to 600 mg/L of sialyllactose.

3. Use according to claim 1, wherein the nutritional composition is an infant formula.

4. Use according to claim 2, wherein the infant formula is in the form of a ready to feed liquid or a powder to be mixed with water before feeding to the infant.

## Patentansprüche

1. Verwendung von Oligofructose und Sialyllactose bei der Herstellung eines Arzneimittels zur Verwendung als Ernährungszusammensetzung für die Ernährung eines Menschen, um bei dem Menschen die Menge von *Bifidobakterien* zu erhöhen und die Bindung pathogener Bakterien zu hemmen, wobei die Ernährungszusammensetzung zur Ernährung eines Menschen bei einer Konzentration dient, die 0,3 g/L bis 6 g/L Oligofructose und 60 mg/L bis 1 g/L Sialyllactose enthält.

2. Verwendung nach Anspruch 1, wobei die Konzentration 1 g/L bis 3 g/L Oligofructose und 100 mg/L bis 600 mg/L Sialyllactose enthält.

3. Verwendung nach Anspruch 1, wobei die Ernährungszusammensetzung eine Säuglingsanfangsnahrung ist.

4. Verwendung nach Anspruch 3, wobei die Säuglingsanfangsnahrung in Form einer zum Füttern fertigen Flüssigkeit oder eines Pulvers vorliegt, das mit Wasser zu mischen ist, bevor der Säugling damit gefüttert wird.

## Revendications

1. Utilisation d'oligofructose et de sialyllactose dans la préparation d'un médicament pour l'utilisation comme composition nutritionnelle pour l'alimentation d'un être humain pour augmenter la quantité de bifidobactéries et inhiber la liaison des bactéries pathogènes chez ledit humain, dans laquelle ladite composition nutritionnelle sert à l'alimentation d'un être humain à une concentration contenant 0,3 g/L à 6 g/L d'oligofructose et 60 mg/L à 1 g/L de sialyllactose.

2. Utilisation selon la revendication 1, dans laquelle ladite concentration contient 1 g/L à 3 g/L d'oligofructose et 100 mg/L à 600 mg/L de sialyllactose.

3. Utilisation selon la revendication 1, dans laquelle la composition nutritionnelle est une préparation pour nourrisson.

4. Utilisation selon la revendication 3, dans laquelle la préparation pour nourrisson se présente sous la forme d'un liquide prêt à absorber ou d'une poudre prête à être mélangée avec de l'eau avant d'être donnée au nourrisson.
